# FASCICULE DE BREVET EUROPEEN

(11) **EP 3 021 770 B1**
(45) Date de publication et mention de la délivrance du brevet: **25.01.2017**
(21) Numéro de dépôt: 14747095.9
(22) Date de dépôt: 11.07.2014
(51) Int. Cl.: A61B 17/70

(54) **DISPOSITIF IMPLANTABLE NOTAMMENT POUR CORRIGER AU MOINS UN NIVEAU VERTÉBRAL**
IMPLANTIERBARE VORRICHTUNG, INSBESONDERE ZUR KORREKTUR VON MINDESTENS EINER VERTEBRALEN EBENE
IMPLANTABLE DEVICE, IN PARTICULAR FOR CORRECTING AT LEAST ONE VERTEBRAL LEVEL

(30) Priorité: 15.07.2013 FR 1356963
(43) Date de publication de la demande: 25.05.2016
(73) Titulaire: Cousin Biotech, 59117 Wervicq Sud (FR)
(72) Inventeur: DENEUVILLERS, Guy, F-62155 Merlimont (FR)
(74) Mandataire: Balesta, Pierre
(86) Numéro de dépôt international: PCT/FR2014/051801
(87) Numéro de publication internationale: WO 2015/007985

(56) Documents cités:
- EP-A1- 2 047 813
- EP-A1- 2 609 882
- FR-A1- 2 750 033
- US-A- 5 899 903

## Description

### Arrière-plan de l'invention

La présente invention concerne le domaine technique des dispositifs implantables, notamment pour corriger au moins un niveau vertébral, éventuellement avec fusion dudit niveau, lesdits dispositifs comprenant une tige, un élément longiligne plat et un moyen de solidarisation dudit élément longiligne à ladite tige.

Les tiges sont utilisées habituellement en combinaison avec un implant pour corriger au moins un niveau vertébral, tels qu'une vis et/ou un crochet recevant ladite tige.

De telles tiges peuvent être mises en oeuvre dans le traitement de la scoliose ou encore le traitement de pathologies dégénératives. On rencontre différents niveaux de dégénérescence, par exemple sur un niveau vertébral comme une discopathie ou sur deux niveaux vertébrales comme le spondylolisthésis ou encore plusieurs niveaux comme la scoliose en C ou en S.

Un exemple de dispositif de fixation vertébrale est décrit dans le document EP 2 609 882 A1. Ce dispositif de fixation vertébrale comprend une tige, un élément longiligne et un moyen de solidarisation dudit élément longiligne dans une position d'enveloppement d'une tige.

Dans ces traitements, la tige peut être mise en liaison avec le niveau vertébral à corriger par l'intermédiaire d'un élément longiligne, lequel passe autour au moins d'une portion d'un corps vertébral et autour de ladite tige puis les extrémités dudit élément longiligne peuvent être maintenues au moyen d'une suture, d'un noeud ou à l'aide d'un moyen de solidarisation implantable. Ledit moyen de solidarisation peut par exemple comprendre un logement recevant la tige et l'élément longiligne à l'état enroulé autour de cette dernière, et des moyens de serrage pour le blocage de la tige et dudit élément longiligne dans ledit logement. Généralement, les moyens de serrage comprennent des moyens de pincement dudit élément longiligne empêchant tout mouvement de ce dernier.

Le pincement dudit élément longiligne a pour inconvénient qu'il peut engendrer une usure prématurée dudit élément longiligne par abrasion, ledit élément longiligne étant généralement dans un textile.

Ces moyens de solidarisation ont également pour inconvénient qu'ils ne permettent pas l'ajustement de la mise en tension de l'élément longiligne, une fois le dispositif implantable posé sur le niveau vertébral à corriger car l'élément longiligne est totalement bloqué par les moyens de pincement. Or, il est souvent nécessaire d'ajuster la correction apportée par la tige, laquelle est en liaison avec l'élément longiligne, une fois le dispositif positionné sur le niveau vertébral à corriger.

En outre ce type de moyen de solidarisation d'un élément longiligne à la tige n'est pas facilement réversible. Il est en effet très complexe de dévisser de multiples vis afin d'ajuster le réglage du niveau vertébral à traiter.

Il existe ainsi un besoin pour un dispositif implantable, notamment pour corriger un niveau vertébral, comprenant un élément longiligne et un moyen de solidarisation dudit élément longiligne dans une position d'enveloppement de ladite tige, permettant audit élément longiligne d'être mise en tension autour de la tige une fois solidarisé dans ladite position d'enveloppement sans séparer le moyen de solidarisation de la tige déjà positionnée sur au moins un niveau vertébral à corriger.

Il existe également un besoin pour un moyen de solidarisation, qui soit facile à mettre en place et à repositionner.

### Objet et résumé de l'invention

La présente invention propose un dispositif implantable selon la revendication indépendante 1. Des réalisations avantageuses de l'invention sont décrites dans les revendications dépendantes.

La présente invention a ainsi pour objet, selon un premier aspect, un dispositif implantable, notamment pour corriger au moins un niveau vertébral, éventuellement avec fusion dudit niveau, comprenant :
a. une tige ayant un rayon externe (Rt) et une circonférence (Ct) déterminées en une zone (z) sur sa longueur (Lₜ) ;
b. un élément longiligne plat ; et
c. un moyen de solidarisation dudit élément longiligne dans une position d'enveloppement partiel de ladite zone (z).

Avantageusement, ledit moyen de solidarisation comprend une première fourche et une seconde fourche ayant une première forme générale partiellement annulaire de rayon interne respectivement ri1 et ri2. Lesdites première et seconde fourches comprennent des faces inférieures d'appui partiellement annulaires délimitant respectivement des première et seconde portions de circonférence supérieures à la moitié de la circonférence (Ct). Ledit moyen de solidarisation comprend une partie d'épaulement agencée par rapport auxdites faces inférieures d'appui des première et seconde fourches en sorte de ménager un passage pour ledit élément longiligne dans ladite position d'enveloppement entre lesdites fourches et ladite partie d'épaulement. Les première et seconde fourches et leurs rayons internes ri1 et ri2 sont déterminés par rapport au rayon externe Rt de la tige en sorte que le placement en force des première et seconde fourches de part et d'autre de l'élément longiligne enveloppant partiellement ladite tige, engendre leur déformation puis leur solidarisation à ladite tige.

Avantageusement, le moyen de solidarisation selon l'invention autorise l'élément longiligne à être mis en tension dans ladite position d'enveloppement puisque l'élément longiligne peut se mouvoir en rotation autour de la tige entre lesdites fourches et la partie d'épaulement. L'élément longiligne est simplement bloqué en translation selon l'axe longitudinal (Lt) de la tige entre lesdites fourches.

Le praticien peut ainsi ajuster la correction du niveau vertébral à traiter quand bien même le dispositif implantable selon l'invention est positionné sur au moins un niveau vertébral à corriger.

Avantageusement, le moyen de solidarisation comprend des première et seconde fourches déformables sous la pression exercée par la tige lors de son placement dans lesdites fourches, puis lesdites fourches se rétractent légèrement et se conforment au diamètre externe Dt de la tige tout en conservant une déformation (dite élastique).

Avantageusement, le moyen de solidarisation selon l'invention se solidarise en clipsant les fourches sur la tige en un emplacement déterminé disposé sur la longueur de celle-ci et qualifiée de zone (z). Le moyen de solidarisation se désolidarise très facilement en exerçant manuellement ou à l'aide d'un outil une traction visant à désengager les fourches de la tige, on obtient alors la déformation des fourches puis leur retrait dans leur position initiale correspondant aux rayons internes ri1 et ri2.

En fonctionnement, l'élément longiligne exerçant une traction dans une direction qui est opposée à la direction selon laquelle la traction de désengagement doit être exercée, le moyen de solidarisation est parfaitement solidarisé à la tige. Cette dernière disposition est une sécurité supplémentaire pour le praticien, le dispositif implantable selon l'invention étant destiné à être implanté sur le rachis.

On comprend au sens de la présente invention par partie d'épaulement tout moyen de liaison assurant la liaison entre les première et seconde fourches et espaçant lesdites première et seconde fourches d'une distance supérieure ou égale à la largeur de l'élément longiligne plat, encore de préférence supérieure ou égale au diamètre externe Dt de la tige.

En fonctionnement, le praticien dispose les première et seconde fourches de part et d'autre de l'élément longiligne disposé dans une position d'enveloppement autour de ladite tige dans une zone (z), en clipsant lesdites fourches sur la tige afin de les déformer pour qu'elles se solidarisent à la tige, leurs rayons internes étant ici supérieurs en fonctionnement ou égaux à celui du rayons externe Rt de la tige.

La déformabilité des première et secondes fourches et leur solidarisation sur ladite tige en sorte qu'il n'y ait ni translation, ni rotation desdites fourches sur la tige dépendent des paramètres suivants : largeur des sections transversales desdites fourches, le ou les matériaux constituant lesdites fourches, les rayons internes ri1 et ri2 déterminés par rapport au rayon externe Rt ; les première et secondes portions circonférentielles des faces inférieures d'appui annulaires déterminées par rapport à la circonférence Ct de la tige en sorte que lesdites fourches enveloppent suffisamment la tige.

L'homme du métier sait ainsi déterminer les largeurs des sections transversales des fourches et le ou les matériaux dans lesquelles elles sont fabriquées afin de leur attribuer une certaine déformabilité (en particulier rester dans une zone élastique de déformation lors de la contrainte exercée pour l'engagement de la tige dans le moyen de solidarisation) et une mémoire de leur forme initiale au repos, correspondant aux rayons internes ri1 et ri2.

Les rayons internes ri1 et ri2 au repos desdites fourches sont déterminés par rapport au rayon externe Rt de la tige en sorte qu'une fois les fourches solidarisées à la tige, ces dernières ne peuvent se déplacer ni en translation ni en rotation sur la tige. De préférence, les rayons internes ri1 et ri2 au repos desdites fourches sont inférieurs ou égaux au rayon externe Rt de la tige, de préférence inférieurs de 3% ou égaux au rayon externe Rt de la tige, encore de préférence inférieurs de 1% ou égaux au rayon externe Rt de la tige, et encore de préférence inférieurs de 0,7% ou égaux au rayon externe de la tige.

L'élément longiligne selon l'invention est de préférence un élément textile, qui peut être tressée, tricotée ou tissée, de préférence tressée.

L'élément longiligne comprend des première et seconde extrémités.

Le dispositif implantable selon l'invention peut également comprendre un dispositif à boucle de fixation tel que décrit dans la demande de brevet EP 2.555.698 A1 au nom de la demanderesse, le dispositif à boucle permettant de solidariser les extrémités de l'élément longiligne entre-elles.
De préférence, le dispositif à boucle de fixation comprend un support rigide comprenant deux parties latérales montées entre des parties avant et arrière, ladite première extrémité étant solidarisée ou apte à être solidarisée à ladite partie arrière et ladite seconde extrémité restant libre.

Le dispositif à boucle de fixation comprend également un élément de blocage monté coulissant sur lesdites parties latérales et délimitant des zones de passage avant et arrière respectivement avec les parties avant et arrière permettant le passage de ladite seconde extrémité libre dans la zone de passage arrière puis la zone de passage avant en enveloppant partiellement ledit élément de blocage en sorte que la portion dudit élément longiligne s'étendant sensiblement entre sa première extrémité et l'élément de blocage forme une boucle de périmètre donné (p) et que l'application de tensions opposées sur les parois internes de la boucle provoque le déplacement dudit élément de blocage vers ladite partie avant et le blocage de la portion d'élément longiligne dans la zone de passage avant entre ledit élément de blocage et la partie avant du support.

Il est également possible de solidariser les première et seconde extrémités de l'élément longiligne entre-elles par l'intermédiaire d'une suture, d'un noeud ou d'une bague métallique à écraser.

De préférence, la distance interne (di) séparant les parties latérales est de l'ordre de celle de la largeur principale (I) dudit élément longiligne ou inférieure.

Le dispositif implantable selon l'invention peut être utilisé dans le traitement des scolioses ou encore dans le traitement de pathologies dégénératives, en particulier dans le traitement de différents niveaux de dégénérescence, par exemple sur un niveau vertébral comme une discopathie ou sur deux niveaux vertébrales comme le spondylolisthésis ou encore plusieurs niveaux comme la scoliose en C ou en S.

L'élément longiligne selon l'invention passe autour au moins d'une portion d'un corps vertébral, par exemple autour d'une apophyse lamaire ou lame vertébrale, ou encore d'une apophyse transversaire et/ou par croisement autour des deux apophyses.

Dans une variante ladite partie d'épaulement est en retrait par rapport auxdites faces inférieures d'appui, respectivement d'une distance h1 et h2 en sorte de ménager un passage pour ledit élément longiligne.

Selon la hauteur h1 et h2, l'élément longiligne peut être plaqué ou non par la partie d'épaulement contre la tige, de préférence les hauteurs h1 et h2 sont supérieures à l'épaisseur (e) de l'élément longiligne en sorte de laisser une certaine liberté de mouvement audit élément longiligne en rotation autour de la tige.

La distance minimum séparant la première fourche de la seconde fourche, correspondant sensiblement à la largeur interne minimum de la partie d'épaulement, combinée aux hauteurs h1 et h2 telles que définies ci-dessus permet avantageusement de ménager un espace pour le passage d'un élément longiligne plat lorsque ledit moyen de solidarisation est solidarisé à la tige. En particulier, la distance séparant la première fourche de la seconde fourche est supérieure ou égale au diamètre externe Dt de la tige en sorte de laisser passer le diamètre de la tige lors de la disposition des première et seconde fourches sur la tige nécessitant un pivotement de 90° desdites fourches.

Dans une variante, le moyen de solidarisation comprend une pièce ayant une face inférieure d'appui apte à recevoir au moins une portion dudit élément longiligne et des moyens de serrage actionnables en translation et agencés par rapport à ladite face inférieure d'appui en sorte que le déplacement en translation desdits moyens de serrage permet le serrage d'au moins une portion dudit élément longiligne contre ladite face inférieure d'appui.

Avantageusement, l'élément longiligne n'est pas serré directement sur la tige mais sur une face inférieure d'appui, de préférence substantiellement plane, par l'intermédiaire des moyens de serrage. La zone de frottement entre l'élément longiligne plat et la face inférieure d'appui est avantageusement plus élevé que si l'élément longiligne avait été pincé ponctuellement de sorte que le serrage et donc la solidarisation d'une portion de l'élément longiligne contre ladite face inférieure est améliorée sans abraser et donc détériorer ladite portion d'élément longiligne.

Dans une sous-variante, ladite pièce comprend au moins une zone agencée en sorte de faire office de premiers organes d'attache apte à coopérer avec des seconds organes d'attache supportés par un outil de préhension, tel un ancillaire, ledit outil aidant au clipsage des fourches sur la tige.

Dans une variante, la partie d'épaulement espace la première fourche et la seconde fourche d'une distance supérieure ou égale à la largeur dudit élément longiligne plat, notamment supérieure ou égale au diamètre externe Dt de la tige.

Dans une variante, la pièce comprend un trou taraudé, et les moyens de serrage comprennent une vis ayant une portion filetée apte à coopérer avec ledit trou taraudé. Lesdits moyens de serrage comprennent en outre une plaque et des moyens de liaison assurant la liaison entre ladite vis et la plaque et agencés en sorte que la mise en rotation de ladite vis provoque le déplacement en translation de ladite plaque pour le serrage d'au moins une portion dudit élément longiligne plat contre la face inférieure d'appui.

Avantageusement, deux portions dudit élément longiligne peuvent être serrées entre la plaque et la face inférieure d'appui. La plaque a une forme substantiellement plane de sorte que ladite au moins une portion dudit élément longiligne est serrée entre la face inférieure d'appui d'une part et la plaque d'autre part qui sont deux surfaces sensiblement planes offrant donc une surface de frottement importante. La surface de contact entre ladite au moins une portion dudit élément longiligne avec la face inférieure d'appui et la plaque étant améliorée, la force de serrage est augmentée. De plus, ladite au moins une portion dudit élément longiligne ne risque pas d'être détériorée par pincement. Un risque d'usure et donc de rupture prématuré dudit élément longiligne sont donc évités.

De préférence, la vis comprend à son extrémité distale une empreinte apte à coopérer avec une empreinte complémentaire supportée par un outil de préhension pour sa mise en rotation, en particulier l'empreinte est de forme polygonale, notamment à six lobes.

Dans une variante, la vis comprend à son extrémité proximale un logement et les moyens de liaison comprennent au moins une portion déformable apte à se déformer pour être insérée dans ledit logement puis à recouvrir sa forme non déformée pour être solidarisée audit logement.

Avantageusement, ladite au moins une portion déformable est déformée pour son introduction dans ledit logement. A l'état non déformée, ladite portion est solidarisée audit logement par blocage dans ce dernier, en particulier ladite portion vient en butée contre différentes régions du logement selon que la vis soit vissée ou dévissée dans ledit trou taraudé.

Ainsi, lors de la mise en rotation de la vis et donc dudit logement, le logement tourne autour de ladite portion déformable et vient en butée contre cette dernière provoquant son déplacement en translation, et corrélativement le déplacement en translation de la plaque puisque les moyens de liaison et donc ladite au moins une portion déformable sont en liaison avec ladite plaque.

Dans une variante, ladite plaque comprend une première face et une seconde face sensiblement planes et opposées, ladite seconde face est orientée vers ladite face inférieure d'appui et ladite première face est orientée vers la vis.

Dans une variante, les moyens de liaison comprennent au moins deux portions déformables, encore de préférence au moins quatre portions déformables. En particulier, lesdites portions déformables forment une queue de sapin déformable.

Dans une variante, les moyens de liaison sont solidarisés à ladite plaque.

Les moyens de liaison sont de préférence solidarisés à la première face de la plaque, notamment la queue de sapin déformable se projette de ladite première face en direction de la vis.

Dans une variante, la pièce a une forme de parallélépipède, notamment de parallélépipède rectangle, comprenant des faces latérales, et des faces supérieure et inférieure, notamment opposées, lesdites première et seconde fourches se projetant selon une première face latérale choisie parmi lesdites faces latérales de la pièce.

Dans une sous-variante, la pièce comprend une ouverture ménagée dans l'une de ses faces latérales faisant offices desdits premiers organes d'attache. De préférence, ladite ouverture a une forme oblongue. De préférence, ladite pièce comprend deux ouvertures, chacune desdites ouvertures étant ménagée dans une de ses faces latérales, lesdites deux faces latérales étant opposées.

De préférence, ladite pièce a une forme de parallélépipède rectangle ou carré, en particulier de cube.

Dans une variante, la partie d'épaulement est la face supérieure de ladite pièce.

Dans une variante, la pièce ayant un volume intérieur comprend un orifice d'admission et un orifice de sortie dans son volume intérieur dudit élément longiligne en sorte de former un passage débouchant au moins sur une zone de serrage délimitée en partie par ladite plaque et la face inférieure d'appui.

Dans une variante, les première et seconde fourches sont en opposition.

En particulier, les première et seconde fourches sont ouvertes l'une sur l'autre en sorte de tourner d'un angle de 90° lors de leur clipsage sur la tige.

Cette disposition évite tout risque de désolidarisation prématurée des première et seconde fourches de la tige en fonctionnement puisque la force nécessaire à la désolidarisation de la première fourche de la tige s'effectue dans une direction qui est opposée à celle nécessaire pour désolidariser la seconde fourche de la tige.

En outre, l'élément longiligne peut envelopper la tige selon une direction perpendiculaire ou non à la direction longitudinale de la tige ce qui offre davantage de possibilité au praticien dans la disposition de l'élément longiligne autour d'au moins une portion d'un corps vertébral.

Cette disposition permet ainsi d'éviter que l'élément longiligne ne monte sur l'une des fourches et reste bien positionné entre les deux fourches ou en butée contre l'une d'elles.

Dans une variante de réalisation, les première et seconde fourches comprennent des premières et secondes extrémités, et la distance entre les premières extrémités d'une part et la distance entre les secondes extrémités d'autre part des première et seconde fourches sont supérieures ou égales à la largeur (I) du élément longiligne plat.

Dans une variante de réalisation, la partie d'épaulement comprend un trou taraudé apte à coopérer avec un outil pourvu d'une portion filetée correspondante.

Il est possible d'engager et de désengager le moyen de solidarisation sur la tige à l'aide dudit outil en liaison avec ledit trou taraudé.

Dans une variante de réalisation, les première et seconde fourches sont dans des plans parallèles.

Optionnellement, l'élément longiligne s'enveloppe ainsi autour de la tige en étant sensiblement perpendiculaire à la direction longitudinale (Lt) de la tige.

Dans une variante de réalisation, les première et seconde fourches sont dans des plans sécants, de préférence formant un angle alpha inférieur ou égal à 45°, de préférence inférieur ou égal à 30°.

Avantageusement, selon la largeur de la lame ou de l'apophyse lamaire, l'élément longiligne ne s'enroule pas dans une direction parfaitement perpendiculaire à la direction longitudinale (Lt) de la tige. En effet, l'élément longiligne peut arriver de côté sur la tige. Cette disposition permet ainsi d'éviter que l'élément longiligne ne monte sur l'une des fourches et reste bien positionné entre les deux fourches ou en butée contre l'une d'elles.

En outre, cette disposition permet de ménager deux points d'appui sur les branches avants des première et seconde fourches qui ne sont pas à l'aplomb des deux points d'appui ménagés au niveau des branches arrières desdites fourches, ce qui améliore la stabilité et le maintien du moyen de solidarisation sur la tige.

Dans une variante de réalisation, le dispositif implantable selon l'invention comprend un moyen de maintien auxiliaire pour sécuriser le moyen de solidarisation solidarisé à ladite zone (z) de ladite tige.

Cette disposition peut être nécessaire lorsque le praticien souhaite une sécurité supplémentaire visant à éviter tout risque de décoaptation du moyen de solidarisation de la tige.

Dans une sous-variante de réalisation, ledit dispositif comprend une vis ayant une portion filetée et ledit moyen de maintien auxiliaire a une forme générale en U inversé ayant une base, présentant une ouverture auxiliaire, de laquelle se projettent transversalement des première et seconde branches pourvues selon leurs extrémités de zones d'appui concaves, ledit moyen auxiliaire est agencé en sorte qu'en fonctionnement les première et seconde branches soient disposées de part et d'autre des première et seconde fourches, ladite ouverture auxiliaire étant au regard dudit trou taraudée, lesdites zones concaves d'appui venant en appui sur la longueur de ladite tige, ladite portion filetée étant passée à travers ladite ouverture et engagée avec ledit trou taraudé.

Le moyen de maintien auxiliaire exerce ainsi une pression sur la partie d'épaulement du moyen de solidarisation par l'intermédiaire de sa base, pression qui s'exerce de manière opposée à la direction selon laquelle peut être exercée la pression de désengagement du moyen de solidarisation.

La pression exercée par le moyen de maintien auxiliaire se répartie ainsi sur sa base et sur les zones d'appui concaves venant en appui sur la tige de part et d'autre des première et seconde fourches.

De préférence, les zones d'appui concaves viennent en appui sur la tige dans une zone dans laquelle les première et seconde fourches ne recouvrent pas la tige.

Dans une variante de réalisation, la vis comprend un épaulement venant en fonctionnement en butée contre la base dudit moyen de maintien auxiliaire.

Avantageusement, selon les hauteurs de la portion filetée et de l'épaulement de la vis, il est possible de comprimer ou non l'élément longiligne par l'intermédiaire de l'extrémité libre de la vis. De préférence, les hauteurs de la portion filetée et de l'épaulement sont déterminées en sorte de ne pas comprimer l'élément longiligne et éviter tout risque de dégradation par piqure et abrasion.

Eventuellement, l'extrémité libre de la vis peut être agencée par rapport à la vis en sorte de ne pas être entraînée en rotation lors du vissage de la vis dans le trou taraudé ménagé dans la partie d'épaulement du moyen de solidarisation. Cette disposition permet ainsi d'exercer simplement une pression sur l'élément longiligne sans rotation sur ce dernier, ce qui limite le risque de détériorer ledit élément longiligne.

De préférence, l'extrémité libre de la vis est plate.

L'ouverture auxiliaire du moyen de maintien auxiliaire peut être éventuellement taraudée afin de coopérer avec la portion filetée de la vis. Dans ce cas, la portion filetée est séparée de l'épaulement par une rainure, la rainure étant en retrait par rapport à l'épaulement et à la portion filetée. Cette disposition permet d'engager le moyen de maintien auxiliaire avec la vis, le moyen de maintien auxiliaire étant bloqué au niveau de la rainure entre l'épaulement et la portion filetée. En fonctionnement, le praticien visse ainsi la portion filetée de la vis dans le trou taraudé de la partie d'épaulement du moyen de solidarisation sans entrainer en rotation le moyen de maintien auxiliaire.

Dans une variante de réalisation, les première et seconde branches dudit moyen de maintien auxiliaire sont dans des plans sécants formant un angle beta, de préférence l'angle béta est inférieur ou égal à 30°, de préférence égal à alpha.

Les première et seconde branches pourraient être également dans des plans sensiblement parallèles, en particulier si les première et seconde fourches sont également dans des plans sensiblement parallèles.

Dans une variante de réalisation, ledit moyen de solidarisation, et éventuellement le moyen de maintien auxiliaire, est dans un ou plusieurs matériaux sélectionné(s) dans la liste comprenant : le titane, l'acier inoxydable, un alliage de titane et de nickel, du polyétheréthercétone (PEEK), PEK (polyéthercétone), chrome cobalt, polyamide 6-6, polyamide 6, polytéréphtalate d'éthylène, et notamment tout autre matériau dit biocompatible à plus de 30 jours.

La présente invention a pour objet selon un deuxième aspect, un kit pour dispositif implantable comprenant un dispositif implantable selon l'une quelconque des variantes de réalisation précédentes, et un outil de préhension dudit moyen de solidarisation comprenant une barre pourvue à son extrémité de premiers organes de fixation aptes à coopérer avec des seconds organes de fixation supportés par ledit moyen de solidarisation.

Dans une sous-variante, les premiers organes de fixation sont un filetage et les seconds organes de fixation sont un trou taraudé ménagé dans la partie d'épaulement ou que comprend la pièce selon l'invention, et éventuellement un taraudage ménagé dans l'ouverture auxiliaire de la base du moyen de maintien auxiliaire.

Selon une alternative, lesdits seconds organes d'attache sont une ou deux ouvertures ménagées dans une ou deux faces latérales de la pièce décrite ci-dessus.

### Brève description des dessins

La présente invention sera mieux comprise à la lecture d'un exemple de réalisation de kit pour dispositif implantable selon l'invention, et d'un second exemple de dispositif implantable selon l'invention cités à titre non limitatif, et illustrés par les figures suivantes dans lesquelles :

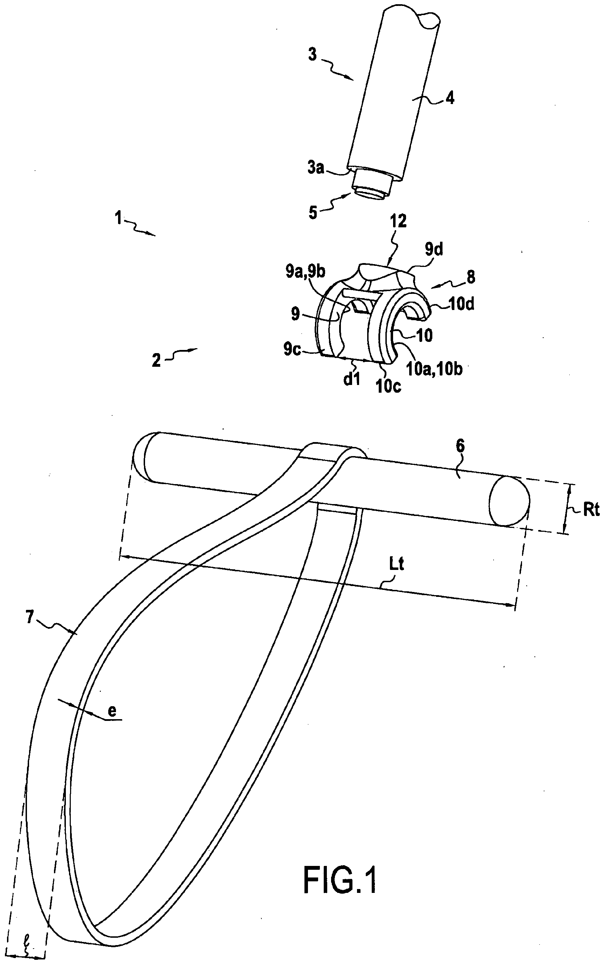

- la figure **1** est une vue en perspective représentant de manière schématique un exemple de kit selon l'invention comprenant un exemple de dispositif implantable selon l'invention ;

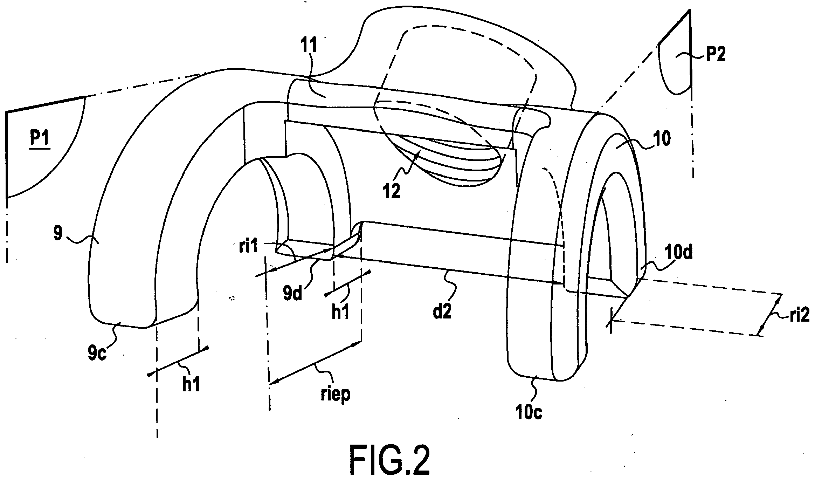

- la figure **2** est une représentation schématique et en perspective du moyen de solidarisation utilisé dans le dispositif implantable représenté à la figure **1** ;
- la figure **3** est une représentation schématique et en perspective d'une variante du moyen de solidarisation représenté à la figure **2** ;
- la figure **4** est une représentation schématique et en perspective d'un moyen de maintien auxiliaire en combinaison avec le dispositif représenté à la figure **1** et comprenant le moyen de solidarisation représenté à la figure **3**;
- la figure **5** est une représentation schématique et en perspective du moyen de maintien auxiliaire représenté à la figure **4** ;
- la figure **6** est une représentation schématique et en perspective de l'exemple de dispositif implantable selon l'invention comprenant le moyen de solidarisation représenté à la figure **3**, une vis et le moyen de maintien auxiliaire représenté aux figures **4** et **5** ;
- la figure **7** est une représentation schématique et en perspective selon une vue antérieure de l'exemple de dispositif implantable représenté à la figure **6** dans laquelle le dispositif comprend en outre un dispositif à boucle de fixation pour la solidarisation du élément longiligne sur lui-même dans ladite position d'enveloppement, le dispositif implantable selon l'invention est représenté dans une position dans laquelle l'élément longiligne passe autour au moins d'une portion d'un corps vertébral, dans cet exemple précis autour d'une apophyse lamaire;
- la figure **8** est une représentation schématique et en perspective selon une vue postérieure du dispositif représenté à la figure **7** ;
- la figure **9** est une représentation schématique et vue de côté, avec des parties arrachées du dispositif représenté aux figures **7** et **8** **;** et
- la figure **10** est une représentation schématique selon une vue postérieure, dans laquelle deux dispositifs implantables selon l'invention tels que ceux représentés en référence aux figures **6** à **9****,** sont positionnés sur au moins un niveau vertébral à corriger.
- la figure **11** est une représentation schématique et en perspective d'un second exemple de dispositif implantable selon l'invention positionné sur au moins un niveau vertébral à corriger ;
- la figure **12** est une représentation schématique et vue de côté des moyens de solidarisation du second exemple de dispositif implantable représenté à la figure **11****;**
- la figure **13** est une représentation schématique et vue de dessus des moyens de solidarisation du second exemple de dispositif implantable représenté à la figure **11**;

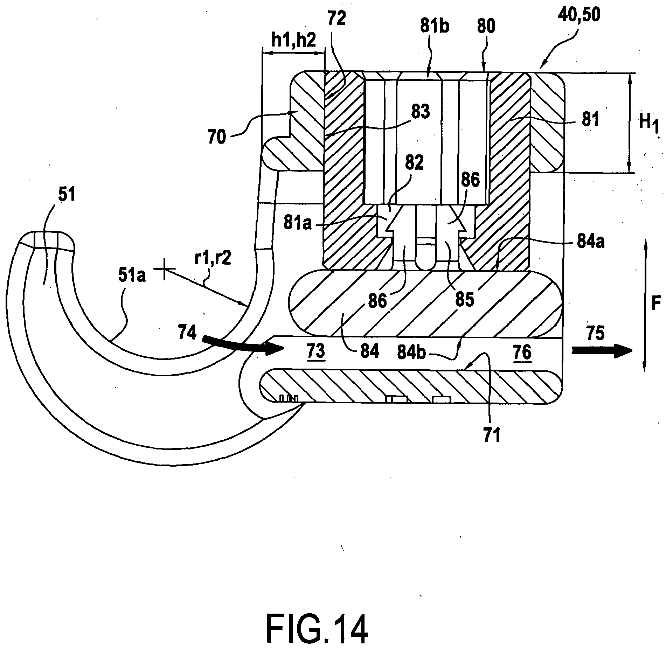

- la figure **14** est une représentation schématique des moyens de solidarisation représentés aux figures **11** à **13** selon le plan de coupe XIV-XIV représenté à la figure **13****.**

### Description détaillée de l'invention

Le kit **1** pour dispositif implantable comprend un exemple de dispositif implantable **2** et un outil de préhension **3** comprenant une barre **4** pourvue à son extrémité **3a** d'un filetage **5.**

Le dispositif implantable **2** comprend une tige **6** ayant un rayon externe **Rt** et une circonférence **Ct** déterminées en une zone **z** sur sa longueur ; un élément longiligne plat **7** ; et un moyen de solidarisation **8** dudit élément longiligne **7** dans une position d'enveloppement partiel de ladite zone **z**. Ledit moyen de solidarisation **8** comprend une première fourche **9** et une seconde fourche **10** ayant une première forme générale partiellement annulaire de rayon interne respectivement **ri1** et **ri2**. Lesdites première **9** et seconde **10** fourches comprennent des faces inférieures d'appui **9a** et **10a** partiellement annulaires délimitant respectivement des première **9b** et seconde **10b** portions de circonférence supérieures à la moitié de la circonférence **Ct.** Ledit moyen de solidarisation **8** comprend une partie d'épaulement **11** ayant une seconde forme partiellement annulaire et coaxiale avec ladite première forme partiellement annulaire, ladite seconde forme ayant un rayon interne riep supérieure aux rayons internes **ri1** et **ri2,** respectivement d'une distance **h1** et **h2** en sorte de ménager un passage pour ledit élément longiligne **7** dans ladite position d'enveloppement entre lesdites fourches **9** et **10** et ladite partie d'épaulement **11.** Les première **9** et seconde **10** fourches et leurs rayons internes **ri1** et **ri2** sont déterminés par rapport au rayon externe **Rt** de la tige **6** en sorte que le placement en force des première **9** et seconde **10** fourches de part et d'autre de l'élément longiligne **7** enveloppant partiellement ladite tige **6,** engendre leur déformation puis leur solidarisation à ladite tige **6.**

Il est également possible de placer le moyen de solidarisation **8** manuellement sur la tige **6** sans l'aide de l'outil de préhension **3**.

Les première **9** et **10** seconde fourches comprennent des premières **9c, 10c** et secondes **9d, 10d** extrémités, et la distance **d1** entre les premières extrémités **9c, 10c** d'une part et la distance **d2** entre les secondes extrémités **9d, 10d** d'autre part des première **9** et seconde **10** fourches sont supérieures ou égales à la largeur **I** de l'élément longiligne plat **7.**

La partie d'épaulement **11** comprend un trou taraudé **12** apte à coopérer avec l'outil **3** pourvu d'une extrémité filetée **3a** correspondante.

Dans cet exemple précis, la première fourche **9** et la seconde fourche **10** sont dans des plans **p1** et **p2** parallèles.

En fonctionnement, le praticien à l'aide de l'outil **3** fait coopérer l'extrémité **3a** filetée avec le trou taraudé **12** ménagé dans la partie d'épaulement **11** en sorte de placer en force les première **9** et seconde **10** fourches sur la zone **z** de la tige **6** engendrant ainsi leur déformation puis leur solidarisation sur la tige **6.** Les fourches **9,10** du moyen de solidarisation **8** sont placées de part et d'autre de l'élément longiligne **7,** lequel est dans une position d'enveloppement de la tige **6.** Le moyen de solidarisation **8** empêche ainsi tout déplacement transversale de l'élément longiligne **7** sur la tige **6,** i.e tout déplacement selon l'axe longitudinale **Lt.**

Il est également possible de positionner le moyen de solidarisation **8** sans l'aide de l'outil **3,** simplement manuellement.

Dans cet exemple précis, les hauteurs **h1** et **h2** sont supérieures à l'épaisseur **e** de l'élément longiligne **7** en sorte d'autoriser des mouvements en rotation de l'élément longiligne **7** autour de la tige **6.**

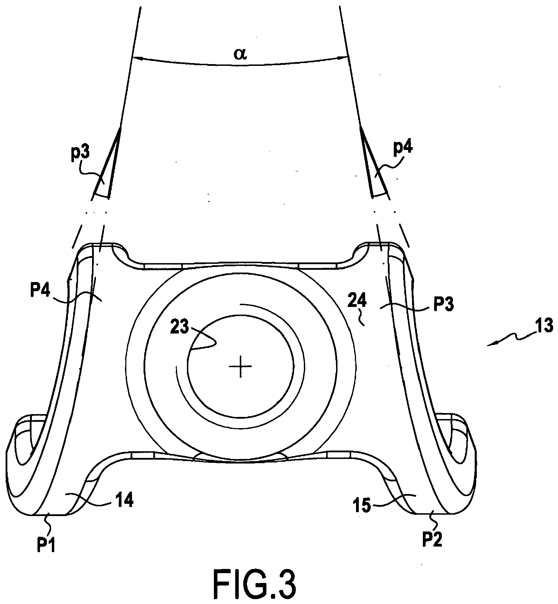

La figure **3** représente une variante **13** du moyen de solidarisation **8** dans laquelle les première **14** et seconde **15** fourches sont dans des plans **p3** et **p4** sécants formant un angle alpha inférieur ou égal à 45°, de préférence inférieur ou égal à 30°. Cette disposition a pour avantage de stabiliser le moyen de solidarisation **13** lorsqu'il est positionné sur la tige **6** puisqu'il développe au moins quatre points d'appui qui ne sont pas à l'aplomb les uns des autres, respectivement les points **P1, P2, P3** et **P4.** En outre, cette disposition évite que l'élément longiligne **7** ne chevauche les première **14** et seconde **15** fourches lorsque l'élément longiligne **7** enveloppe la tige **6** dans une direction qui n'est pas sensiblement perpendiculaire à l'axe **Lt** de la tige **6.**

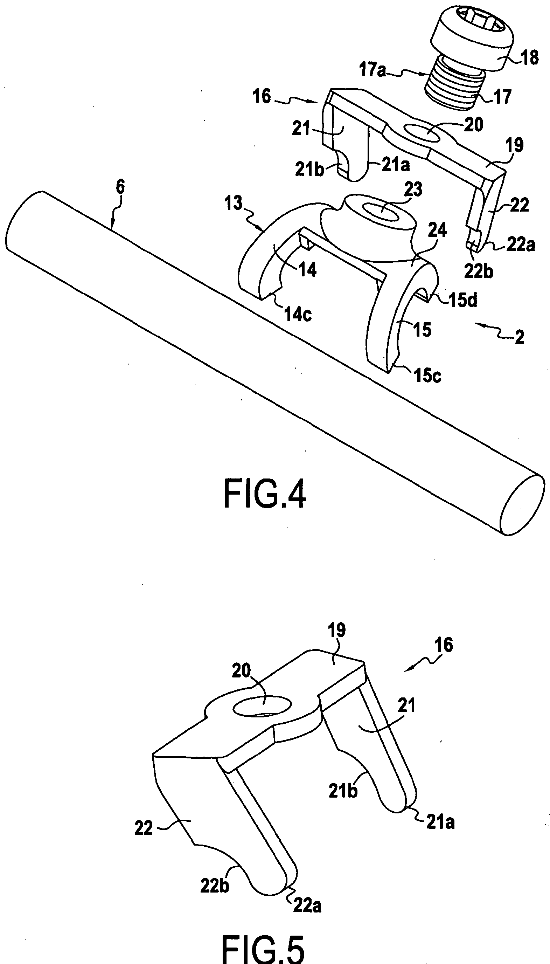

Le dispositif implantable **2** peut comprendre également un moyen de maintien auxiliaire **16** pour sécuriser le moyen de solidarisation **13** solidarisé à ladite zone **z** de ladite tige **6** tel que représenté aux figures **4** à **6****.**

Le dispositif implantable **2** comprend également une vis **17** ayant une portion filetée **17**a et un épaulement **18.** Le moyen de maintien auxiliaire **16** a une forme générale en U inversé ayant une base **19,** présentant une ouverture auxiliaire **20,** de laquelle se projettent transversalement des première **21** et seconde **22** branches pourvues selon leurs extrémités **21a** et **22a** de zones d'appui concaves **21b** et **22b.** Le moyen auxiliaire **16** est agencé en sorte qu'en fonctionnement les première **21** et seconde **22** branches soient disposées de part et d'autre des première **14** et seconde **15** fourches, ladite ouverture auxiliaire **20** étant au regard du trou taraudé **23,** lesdites zones concaves d'appui **21b** et **22b** venant en appui sur la longueur de ladite tige **6.** La portion filetée **17a** de la vis **17** est alors passée à travers l'ouverture auxiliaire **20** et engagée avec ledit trou taraudé **23** jusqu'à ce que l'épaulement **18** vienne en butée contre la partie d'épaulement **24,** en particulier contre le trou taraudé **23** se projetant dans cet exemple précis de la partie d'épaulement **24.** Selon la hauteur de la portion filetée **17a** de la vis **17,** il est possible de pincer ou non l'élément longiligne **7** passant entre les fourches **14** et **15.**

De préférence, la hauteur de la portion filetée **17a** de la vis **17** est déterminée en sorte de ne pas pincer l'élément longiligne **7** et lui laisser une possibilité de mouvement en rotation autour de la tige **6.**

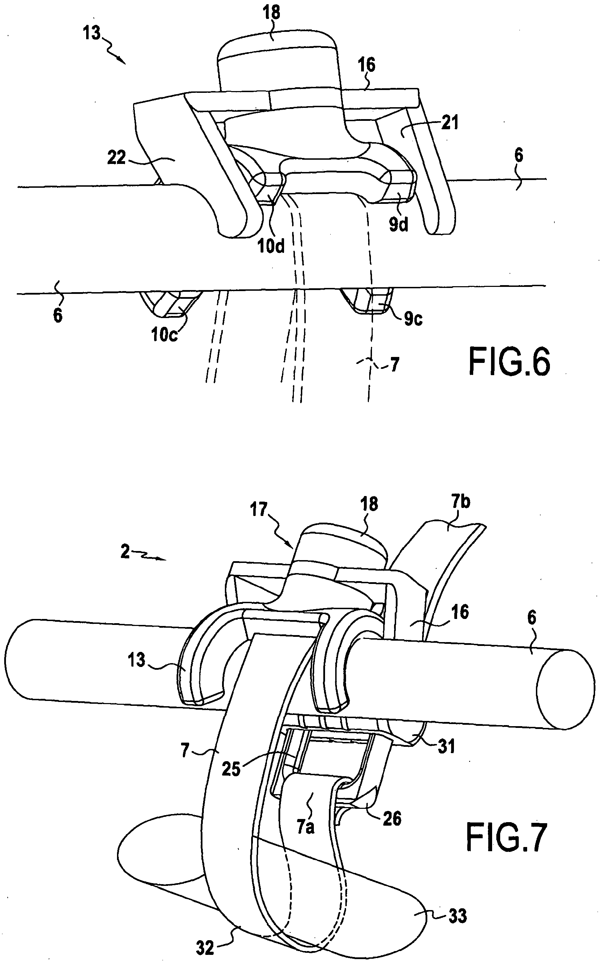

Le dispositif implantable **2** peut également comprendre, tel que représenté aux figures **7** à **9****,** un dispositif à boucle de fixation **25,** tel que décrit dans la demande de brevet EP 2.555.698 A1.

De préférence, ce dispositif à boucle de fixation **25** comprend ainsi un support rigide **26** comprenant deux parties latérales **27** et **28** montées entre des parties avant **29** et arrière **30,** la première extrémité **7a** de l'élément longiligne **7** étant solidarisée ou apte à être solidarisée à ladite partie arrière **30** et la seconde extrémité **7b** de l'élément longiligne **7** restant libre.

Le dispositif à boucle de fixation **25** comprend un élément de blocage **31** monté coulissant sur lesdites parties latérales **27** et **28** et délimitant des zones de passage avant et arrière respectivement avec les parties avant **29** et arrière **30** permettant le passage de ladite seconde extrémité libre **7b** dans la zone de passage arrière puis la zone de passage avant en enveloppant partiellement ledit élément de blocage **31** en sorte que la portion dudit élément longiligne **7,** s'étendant sensiblement entre sa première extrémité **7a** et l'élément de blocage **31,** forme une boucle **32** de périmètre donné **p** et l'application de tensions opposées sur les parois internes de la boucle **32** provoque le déplacement dudit élément de blocage **31** vers ladite partie avant **29** et le blocage de la portion d'élément longiligne **7** dans la zone de passage avant entre ledit élément de blocage **31** et la partie avant **29** du support **26.**

De préférence, la distance interne di séparant les parties latérales **27** et **28** est de l'ordre de celle de la largeur principale **I** dudit élément longiligne **7.**

Les première **7a** et seconde **7b** extrémités de l'élément longiligne **7** sont solidarisées entre-elles grâce au dispositif à boucle de fixation **25.**

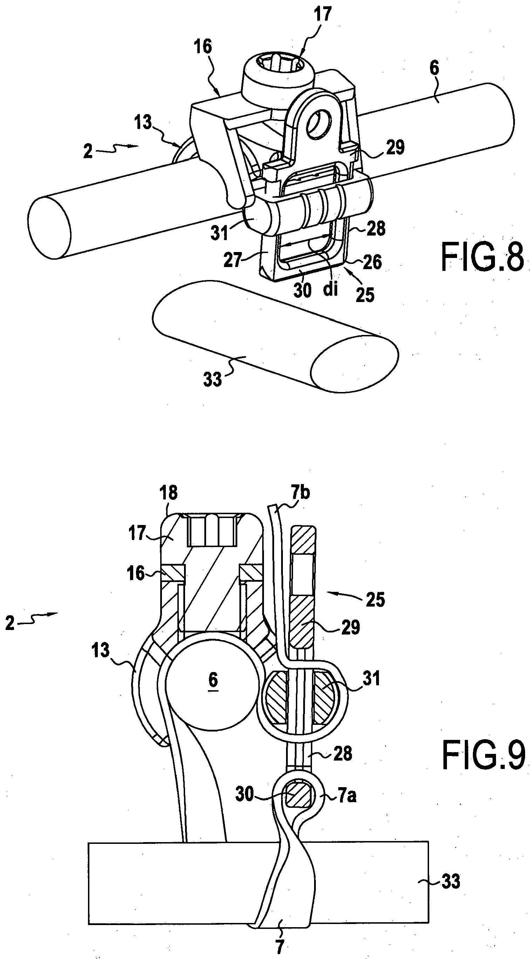

Sur les figures **7** et **9****,** l'élément longiligne est dans une position d'enveloppement partiel de la tige **6** et passe également autour d'une portion du corps vertébral **33,** dans cet exemple précis autour d'une apophyse lamaire. L'élément longiligne **7** n'a pas été représenté à la figure **8** pour des raisons de simplification.

En fonctionnement, le praticien ajuste très facilement la mise en tension de l'élément longiligne **7 ;** il lui suffit de débloquer l'élément de blocage **31,** puis d'exercer une traction sur la seconde extrémité libre **7b** de l'élément longiligne **7 ;** l'élément longiligne **7** coulisse alors autour de la tige **6,** entre les fourches **14** et **15** et la partie d'épaulement **24,** et ce sans désolidariser le moyen de solidarisation **13.** Puis le praticien bloque à nouveau l'élément longiligne en rotation autour de la tige **6,** par l'intermédiaire de l'élément de blocage **31** en le faisant coulisser vers la partie avant **29.**

Les rayons internes des première **9, 14** et seconde **10, 15** fourches sont agencés par rapport au rayon externe **Rt** de la tige **6** en sorte qu'une fois le moyen de solidarisation **8, 13** clipsé sur la tige **6,** ce dernier ne puisse se déplacer ni en rotation, ni en translation selon l'axe longitudinal **Lt** de la tige **6.**

Dans cet exemple précis, Les rayons internes des première **9, 14** et seconde **10,15** fourches sont inférieurs de 0,7% par rapport au rayon externe **Rt** de la tige **6 ;** de préférence, lesdits rayons internes sont de 2,73 mm et le rayon externe **Rt** est de 2,75 mm.

Dans cet exemple précis, la largeur des sections transversales des premières extrémités **9c, 10c** et **14c** et **15c** des premières **9, 14** et secondes **10, 15** fourches est de 1,50 mm, et la largeur des sections transversales des secondes extrémités **9d, 10d** et **14d, 15d** des premières **9, 14** et secondes **10, 15** fourches est de 2,00 mm.

La distance **d1** séparant les premières extrémités **9c** et **10c** d'une part, et **14c** et **15c** d'autre part, est de 10 mm. La distance **d2** séparant les secondes extrémités **9d** et **10d** d'une part, et **14d** et **15d** d'autre part, est de 15 mm.

De préférence, le moyen de solidarisation **8** ou **16** est en titane.

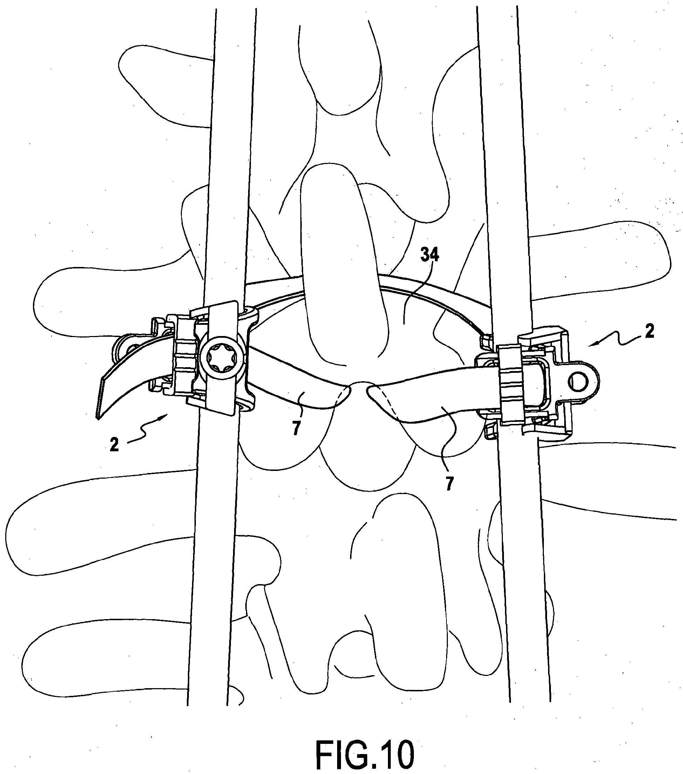

La figure **10** représente deux dispositifs implantables **2** selon l'invention disposés sur au moins un niveau vertébral à corriger.

Les deux éléments longilignes **7** sont ainsi passés autour de portions d'un corps vertébral **34,** dans cet exemple précis autour des apophyses lamaires.

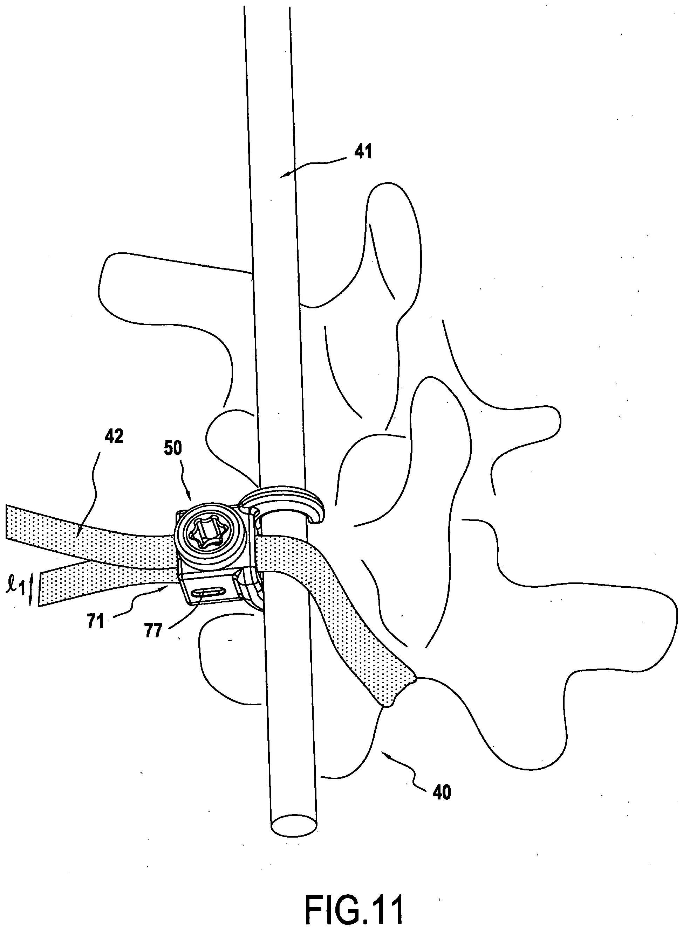

Le dispositif implantable **40** représenté à la figure **11** sur au moins un niveau vertébral à corriger comprend une tige **41** ayant un rayon externe **Rt** et une circonférence **Ct** déterminées en une zone **z** sur sa longueur ; un élément longiligne plat **42 ;** et un moyen de solidarisation **50** dudit élément longiligne **42** dans une position d'enveloppement partiel de ladite zone **z.** Ledit moyen de solidarisation **50** comprend une première fourche **51** et une seconde fourche **52** ayant une première forme générale partiellement annulaire de rayon interne respectivement **ri1** et **ri2.** Lesdites première **51** et seconde **52** fourches comprennent des faces inférieures d'appui **51a** et **52a** partiellement annulaires délimitant respectivement des première **51b** et seconde **52b** portions de circonférence supérieures à la moitié de la circonférence **Ct.** Ledit moyen de solidarisation **50** comprend une partie d'épaulement **60** agencée par rapport auxdites faces inférieures d'appui **51a,52a** des première et seconde fourches **51,52** en sorte de ménager un passage pour ledit élément longiligne **42** dans ladite position d'enveloppement entre lesdites fourches (**51,52**) et ladite partie d'épaulement **60.** En particulier, ladite partie d'épaulement **60** est en retrait par rapport auxdites faces inférieures d'appui **51a,52a,** respectivement d'une distance **h1** et **h2** en sorte de ménager un passage pour ledit élément longiligne **42.** Les première **51** et seconde **52** fourches et leurs rayons internes **ri1** et **ri2** sont déterminés par rapport au rayon externe **Rt** de la tige **42** en sorte que le placement en force des première **51** et seconde **52** fourches de part et d'autre de l'élément longiligne **42** enveloppant partiellement ladite tige **41,** engendre leur déformation puis leur solidarisation à ladite tige **41.**

Le moyen de solidarisation **50** comprend une pièce **70** ayant une face inférieure d'appui **71** apte à recevoir au moins une portion dudit élément longiligne **42** et des moyens de serrage **80** actionnables en translation et agencés par rapport à ladite face inférieure d'appui **71** en sorte que le déplacement en translation desdits moyens de serrage **80** permet le serrage d'au moins une portion dudit élément longiligne **42** contre ladite face inférieure d'appui **71.** Tel que cela est visible à la figure **11****,** l'élément longiligne **42** passe autour au moins une portion d'un corps vertébral puis ses deux extrémités libres passent sur la face inférieure d'appui **71** en sorte que les deux portions superposées dudit élément longiligne **42** soient maintenues ensemble et serrées contre ladite face inférieure d'appui **71** par les moyens de serrage **80.**

La partie d'épaulement **60** espace les première **51** et seconde **52** fourches d'une distance **d3** supérieure ou égale à la largeur **I1** dudit élément longiligne plat **42** et supérieure ou égale au diamètre Dt de la tige **41 afin** que les fourches **51,52** puissent pivoter de 90° lors de leur placement sur la tige **41.**

La pièce **70** comprend un trou taraudé **72,** et les moyens de serrage **80** comprennent une vis **81** ayant une portion filetée **83** apte à coopérer avec ledit trou taraudé **72** sur la hauteur **H1.** Les moyens de serrage **80** comprennent en outre une plaque **84** et des moyens de liaison **85** assurant la liaison entre ladite vis **81** et la plaque **84** et agencés en sorte que la mise en rotation de ladite vis **81** provoque le déplacement en translation selon l'axe **F** de ladite plaque **84** pour le serrage d'au moins une portion dudit élément longiligne **42** contre la face inférieure d'appui **71.** La plaque **84** comprend une première face **84a** et une seconde face **84b** qui sont sensiblement planes et opposées. La vis **81** comprend à son extrémité proximale **81**a un logement **82** et à son extrémité distale **81b** une empreinte **87,** notamment une empreinte polygonale, en particulier à six lobes, apte à coopérer avec l'empreinte complémentaire d'un outil de préhension pour actionner les moyens de serrage **80.**

Les moyens de liaison **85** comprennent au moins une portion déformable **86** apte à se déformer pour être insérée dans ledit logement **82** puis à recouvrir sa forme non déformée pour être solidarisée audit logement **82.** Dans cet exemple précis, les moyens de liaison **85** comprennent quatre portions déformables distinctes **86** (dont seulement deux sont représentées sur le plan de coupe XIV-XIV) formant une queue de sapin déformable. Les moyens de liaison **85** sont dans cet exemple précis solidarisés à la plaque **84,** en particulier à sa première face **84a** sensiblement plane.

La pièce **70** a dans cet exemple précis une forme de parallélépipède, notamment de parallélépipède rectangle, en particulier carré, comprenant des faces latérales (**70c, 70d, 70e, 70f),** et des faces supérieure **70a** et inférieure **70b,** notamment opposées, lesdites première **51** et seconde **52** fourches se projetant selon une première face latérale **70c** choisie parmi lesdites faces latérales **70c, 70d, 70e, 70f** de la pièce **70.** La partie d'épaulement **60** est la face supérieure **70a** de ladite pièce **70.** La pièce **70** ayant un volume intérieur **73** comprend un orifice d'admission **74** et un orifice de sortie **75** dans son volume intérieur **73** dudit élément longiligne **42** en sorte de former un passage débouchant au moins sur une zone de serrage **76** délimitée en partie par ladite plaque **84** et la face inférieure d'appui **71.**

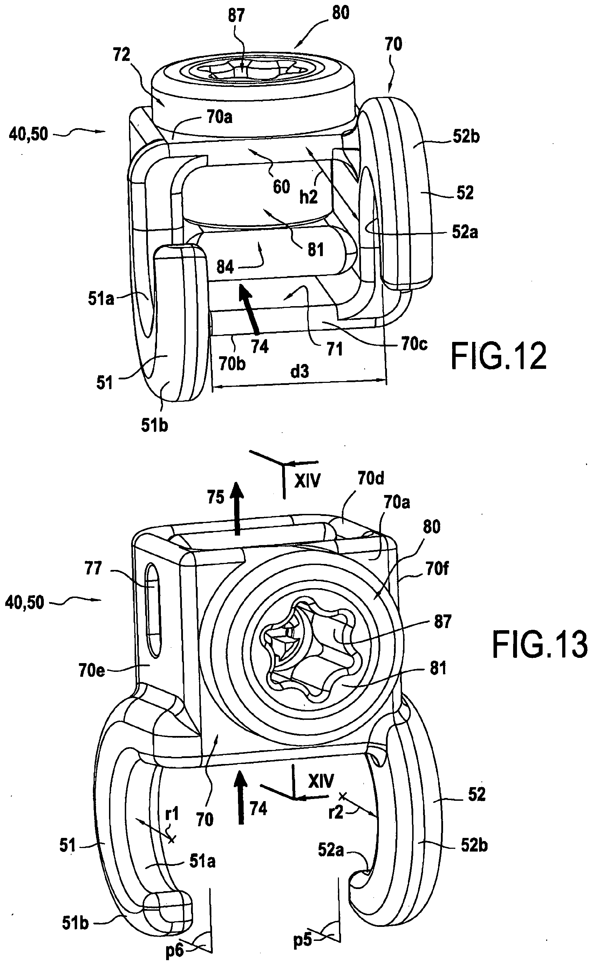

La pièce **70** comprend des premiers organes d'attache **77** disposés dans ses faces latérales **70e** et **70f,** en particulier deux ouvertures, notamment de forme oblongue, seule l'ouverture ménagée dans la face latérale **70e** est représentée aux figures **11** et **13****.** La seconde ouverture, non visible, est disposée dans la face latérale **70f**, opposée à la face latérale **70e.** Les premiers organes d'attache **77** sont aptes à coopérer avec des seconds organes d'attache (non représentés) d'un outil de préhension, ledit outil aidant au placement des moyens de solidarisation **50,** et donc des fourches **51,52** sur la tige **41.**

Les première **51** et seconde **52** fourches sont en opposition et dans des plans parallèles **p5, p6.**

En fonctionnement, une fois l'élément longiligne **42** placé autour d'au moins une portion d'un corps vertébral et autour au moins partiellement de la tige **41,** le praticien à l'aide d'un outil pourvu des seconds organes d'attache coopérant avec les premiers organes d'attache **77** place en force les moyens de solidarisation **50** sur la tige **41** en faisant pivoter les première **51** et seconde fourche **52** d'environ 90° autour de la tige **41** engendrant ainsi leur déformation puis leur solidarisation à la tige **41** sur la zone **z.** La disposition des moyens de solidarisation **50** peut être effectuée également manuellement par le praticien. Dans les deux cas, cette opération provoque la déformation des fourches **51,52** puis leur solidarisation sur ladite tige **41.** Le praticien passe ensuite les. extrémités libres de l'élément longiligne plat **42** dans la zone de serrage **76** à travers l'orifice d'entrée **74** et l'orifice de sortie **75** et exerce une tension manuellement ou une tension à l'aide d'un outil approprié sur les extrémités libres de l'élément longiligne **42.** Enfin, le praticien actionne les moyens de serrage **80** par l'intermédiaire d'un outil de préhension (non représenté) pourvu d'une empreinte complémentaire de l'empreinte **87** ménagée dans la partie proximale **81b** de la vis **81** pour la mise en rotation de la vis **81.** Le logement **82** vient alors en butée contre les parties supérieures desdites portions déformables **86** entrainant ainsi en translation selon l'axe **F** la plaque **84** pour la rapprocher de la face inférieure d'appui **71** et ainsi serrer les portions superposées de l'élément longiligne **42.**

Avantageusement, la disposition en opposition des première et seconde fourches **51,52** évite toute désolidarisation desdites fourches **51,52** de la tige **41.** En outre, la disposition d'au moins une portion de l'élément longiligne **42,** dans cet exemple précis d'au moins deux portions de l'élément longiligne **42** superposées entre la seconde face sensiblement plane **84b** de la plaque **84** et la face inférieure d'appui **71**, également sensiblement plane, permet une surface de contact importante avec l'élément longiligne **42** et ainsi une force de serrage très importante. A titre d'exemple, pour un couple de 6N.m (newtons.mètre) exercé sur la vis **81** par l'intermédiaire d'un outil de préhension, sous l'effet d'une traction de 1067 newtons exercée sur les deux extrémités libres. de l'élément longiligne **42** passé dans les moyens de solidarisation **50,** l'élément longiligne **42** reste parfaitement maintenu entre la plaque **84** et la face inférieure d'appui **71.**

Enfin, les fourches **51,52** du moyen de solidarisation **50** sont placées de part et d'autre de l'élément longiligne **42,** lequel est dans une position d'enveloppement de la tige **41.** Le moyen de solidarisation **50** empêche ainsi tout déplacement transversale de l'élément longiligne **42** sur la tige **41,** i.e tout déplacement selon l'axe longitudinale **Lt.**

## Revendications

1. Dispositif implantable (2,40), notamment pour corriger au moins un niveau vertébral, éventuellement avec fusion dudit niveau, comprenant :
a. une tige (6,41) ayant un rayon externe (Rt) et une circonférence (Ct) déterminées en une zone (z) sur sa longueur (Lₜ) ;
b. un élément longiligne plat (7,42) ;
c. un moyen de solidarisation (8,13,50) dudit élément longiligne (7,42) dans une position d'enveloppement partiel de ladite zone (z) ;
**caractérisé en ce que** ledit moyen de solidarisation (8,13,50) comprend une première fourche (9,14,51) et une seconde fourche (10,15,52) ayant une première forme générale partiellement annulaire de rayon interne respectivement ri1 et ri2, **en ce que** lesdites première (9,14,51) et seconde fourches (10,15,52) comprennent des faces inférieures d'appui (9a,10a,51a,52a) partiellement annulaires délimitant respectivement des première (9b,51b) et seconde (10b,52b) portions de circonférence supérieures à la moitié de la circonférence (Ct), **en ce que** ledit moyen de solidarisation (8,13,50) comprend une partie d'épaulement (11,24,60) agencée par rapport auxdites faces inférieures d'appui (9a,10a,51a,52a) des première et seconde fourches (9,14,51,10,15,52) en sorte de ménager un passage pour ledit élément longiligne dans ladite position d'enveloppement entre lesdites fourches et ladite partie d'épaulement, et **en ce que** les première (9,14,51) et seconde (10,15,52) fourches et leurs rayons internes ri1 et ri2 sont déterminés par rapport au rayon externe Rt de la tige (6,41) en sorte que le placement en force des première (9,14,51) et seconde (10,15,52) fourches de part et d'autre de l'élément longiligne (7,42) enveloppant partiellement ladite tige (6,7), engendre leur déformation puis leur solidarisation à ladite tige (6,7).

2. Dispositif implantable (2,40) selon la revendication 1, **caractérisé en ce que** ladite partie d'épaulement (11,24,60) est en retrait par rapport auxdites faces inférieures d'appui (9a,10a,51a,52a), respectivement d'une distance h1 et h2 en sorte de ménager un passage pour ledit élément longiligne (7,42).

3. Dispositif implantable (40) selon l'une ou l'autre des revendications 1 et 2, **caractérisé en ce que** le moyen de solidarisation (50) comprend une pièce (70) ayant une face inférieure d'appui (71) apte à recevoir au moins une portion dudit élément longiligne (42) et des moyens de serrage (80) actionnables en translation et agencés par rapport à ladite face inférieure d'appui (71) en sorte que le déplacement en translation desdits moyens de serrage (80) permet le serrage d'au moins une portion dudit élément longiligne (42) contre ladite face inférieure d'appui (71).

4. Dispositif implantable (2,40) selon l'une quelconque des revendications 1 à 3, **caractérisé en ce que** la partie d'épaulement (11,24,60) espace la première fourche (9,14,51) et la seconde fourche (10,15,52) d'une distance (d1,d2,d3) supérieure ou égale à la largeur (I,I1) dudit élément longiligne plat (7,42).

5. Dispositif implantable (40) selon l'une ou l'autre des revendications 3 et 4, **caractérisé en ce que** la pièce (70) comprend un trou taraudé (72), et les moyens de serrage (80) comprennent une vis (81) ayant une portion filetée (83) apte à coopérer avec ledit trou taraudé (72), et **en ce que** lesdits moyens de serrage (80) comprennent en outre une plaque (84) et des moyens de liaison (85) assurant la liaison entre ladite vis (81) et la plaque (84) et agencés en sorte que la mise en rotation de ladite vis (81) provoque le déplacement en translation de ladite plaque (84) pour le serrage d'au moins une portion dudit élément longiligne (42) contre la face inférieure d'appui (71).

6. Dispositif implantable (40) selon la revendication 5, **caractérisé en ce que** la vis (81) comprend à son extrémité proximale (81a) un logement (82) et **en ce que** les moyens de liaison (85) comprennent au moins une portion déformable (86) apte à se déformer pour être insérée dans ledit logement (82) puis à recouvrir sa forme non déformée pour être solidarisée audit logement (82).

7. Dispositif implantable (40) selon l'une ou l'autre des revendications 5 et 6, **caractérisé en ce que** les moyens de liaison (85) sont solidarisés à ladite plaque (84).

8. Dispositif implantable (40) selon l'une quelconque des revendications 3 à 7, **caractérisé en ce que** la pièce (70) a une forme de parallélépipède, notamment de parallélépipède rectangle, comprenant des faces latérales (70c,70d,70e,70f), et des faces supérieure (70a) et inférieure (70b), notamment opposées, lesdites première (51) et seconde (52) fourches se projetant selon une première face (70c) latérale choisie parmi lesdites faces latérales (70c,70f) de la pièce (70).

9. Dispositif implantable (40) selon la revendication 8, **caractérisé en ce que** la partie d'épaulement (60) est la face supérieure (70a) de ladite pièce (70).

10. Dispositif implantable (40) selon l'une quelconque des revendications 3 à 9, **caractérisé en ce que** la pièce (70) ayant un volume intérieur (73) comprend un orifice d'admission (74) et un orifice de sortie (75) dans son volume intérieur (73) dudit élément longiligne (42) en sorte de former un passage débouchant au moins sur une zone de serrage (76) délimitée en partie par ladite plaque (84) et la face inférieure d'appui (71).

11. Dispositif implantable (40) selon l'une quelconque des revendications 1 à 10, **caractérisé en ce que** les première (51) et seconde (52) fourches sont en opposition.

12. Dispositif implantable (2,40) selon l'une quelconque des revendications 1 à 11, **caractérisé en ce que** les première (9,51) et seconde (10,52) fourches sont dans des plans parallèles (p1,p2,p5,p6).

13. Dispositif implantable (2,40) selon l'une quelconque des revendications 1 à 12, **caractérisé en ce que** ledit moyen de solidarisation (8,13,50), est dans un ou plusieurs
matériaux sélectionné(s) dans la liste comprenant : le titane, l'acier inoxydable, un alliage de titane et de nickel, du polyétheréthercétone (PEEK), PEK (polyéthercétone), chrome cobalt, polyamide 6-6, polyamide 6, polytéréphtalate d'éthylène.

14. Kit (1) pour dispositif implantable comprenant un dispositif implantable (2,40) selon l'une quelconque des revendications 1 à 13, et un outil de préhension (3) dudit moyen de solidarisation (8,13,40) comprenant une barre (4) pourvue à son extrémité (3a) de premiers organes de fixation (5) aptes à coopérer avec des seconds organes de fixation (12, 23) supportés par ledit moyen de solidarisation (8,13,40).

## Patentansprüche

1. Implantierbare Vorrichtung (2, 40), insbesondere zum Korrigieren wenigstens einer Wirbelebene, eventuell mit Verschmelzen der Ebene, umfassend:
a. eine Stange (6, 41) mit einem bestimmten Außenradius (Rt) und einem bestimmten Umfang (Ct) in einem Bereich (z) ihrer Länge (Lₜ),
b. ein flaches langgestrecktes Element (7, 42),
c. ein Mittel zum festen Verbinden (8, 13, 50) des langgestreckten Elements (7, 42) in einer Position eines teilweisen Umfassens des Bereichs (z),
**dadurch gekennzeichnet, dass** das Mittel zum festen Verbinden (8, 13, 50) eine erste Gabel (9, 14, 51) und eine zweite Gabel (10, 15, 52), die eine teilweise ringförmige erste Gesamtform mit einem Innenradius ri1 bzw. ri2 aufweisen, umfasst, dass die erste (9, 14, 51) und die zweite (10, 15, 52) Gabel teilweise ringförmige untere Anlageseiten (9a, 10a, 51 a, 52a) umfassen, welche einen ersten (9b, 51 b) bzw. einen zweiten (10b, 52b) Umfangsabschnitt größer als die Hälfte des Umfangs (Ct) begrenzen, dass das Mittel zum festen Verbinden (8, 13, 50) einen Schulterteil (11, 24, 60) umfasst, welcher gegenüber den unteren Anlageseiten (9a, 10a, 51a, 52a) der ersten und der zweiten Gabel (9, 14, 51, 10, 15, 52) so angeordnet ist, dass ein Durchgang für das langgestreckte Element in der Umfassungsposition zwischen den Gabeln und dem Schulterteil ausgespart wird, und dass die erste (9, 14, 51) und die zweite (10, 15, 52) Gabel und ihre Innenradien ri1 und ri2 in Bezug auf den Außenradius Rt der Stange (6, 41) derart festgelegt sind, dass das Anordnen mit Kraft der ersten (9, 14, 51) und der zweiten (10, 15, 52) Gabel auf beiden Seiten des die Stange (6, 7) teilweise umgreifenden langgestreckten Elements (7, 42) deren Verformung, dann deren festes Verbinden mit der Stange (6, 7) bewirkt.

2. Implantierbare Vorrichtung (2, 40) nach Anspruch 1, **dadurch gekennzeichnet, dass** der Schulterteil (11, 24, 60) gegenüber den unteren Anlageseiten (9a, 10a, 51 a, 52a) um einen Abstand h1 bzw. h2 zurückspringt, um einen Durchgang für das langgestreckte Element (7, 42) auszusparen.

3. Implantierbare Vorrichtung (40) nach dem einen oder dem anderen der Ansprüche 1 und 2, **dadurch gekennzeichnet, dass** das Mittel zum festen Verbinden (50) ein Teil (70) mit einer unteren Anlageseite (71), die geeignet ist, wenigstens einen Abschnitt des langgestreckten Elements (42) aufzunehmen, und Klemmmittel (80) umfasst, die verschieblich betätigbar und gegenüber der unteren Anlageseite (71) so angeordnet sind, dass die Verschiebebewegung der Klemmmittel (80) das Festklemmen wenigstens eines Abschnitts des langgestreckten Elements (42) an der unteren Anlageseite (71) ermöglicht.

4. Implantierbare Vorrichtung (2, 40) nach einem der Ansprüche 1 bis 3, **dadurch gekennzeichnet, dass** der Schulterteil (11, 24, 60) die erste Gabel (9,14, 51) und die zweite Gabel (10, 15, 52) um einen Abstand (d1, d2, d3) beabstandet, der größer als die oder gleich der Breite (I, I1) des flachen langgestreckten Elements (7, 42) ist.

5. Implantierbare Vorrichtung (40) nach dem einen oder dem anderen der Ansprüche 3 und 4, **dadurch gekennzeichnet, dass** das Teil (70) eine Gewindebohrung (72) aufweist und die Klemmmittel (80) eine Schraube (81) mit einem Gewindeabschnitt (83), der geeignet ist, mit der Gewindebohrung (72) zusammenzuwirken, umfassen, und dass die Klemmmittel (80) ferner eine Platte (84) und Verbindungsmittel (85) umfassen, welche die Verbindung zwischen der Schraube (81) und der Platte (84) sicherstellen und derart angeordnet sind, dass das Indrehungversetzen der Schraube (81) die Verschiebebewegung der Platte (84) für das Festklemmen wenigstens eines Abschnitts des langgestreckten Elements (42) an der unteren Anlageseite (71) bewirkt.

6. Implantierbare Vorrichtung (40) nach Anspruch 5, **dadurch gekennzeichnet, dass** die Schraube (81) an ihrem proximalen Ende (81a) eine Aufnahme (82) umfasst und dass die Verbindungsmittel (85) wenigstens einen deformierbaren Abschnitt (86) umfassen, der geeignet ist, sich zu verformen, um in die Aufnahme (82) eingefügt zu werden, dann wieder seine nicht deformierte Form anzunehmen, um mit der Aufnahme (82) fest verbunden zu werden.

7. Implantierbare Vorrichtung (40) nach dem einen oder dem anderen der Ansprüche 5 und 6, **dadurch gekennzeichnet, dass** die Verbindungsmittel (85) mit der Platte (84) fest verbunden sind.

8. Implantierbare Vorrichtung (40) nach einem der Ansprüche 3 bis 7, **dadurch gekennzeichnet, dass** das Teil (70) eine Parallelepiped-Form, insbesondere eine Quader-Form aufweist, mit Seitenflächen (70c, 70d, 70e, 70f) und einer Oberseite (70a) und eine Unterseite (70b), die insbesondere gegenüberliegen, wobei die erste (51) und die zweite (52) Gabel sich entlang einer aus den Seitenflächen (70c, 70f) des Teils (70) ausgewählten ersten Seitenfläche (70c) erstrecken.

9. Implantierbare Vorrichtung (40) nach Anspruch 8, **dadurch gekennzeichnet, dass** der Schulterteil (60) die Oberseite (70a) des Teils (70) ist.

10. Implantierbare Vorrichtung (40) nach einem der Ansprüche 3 bis 9, **dadurch gekennzeichnet, dass** das Teil (70), das einen Innenraum (73) aufweist, eine Öffnung zum Zuführen (74) und eine Öffnung für den Austritt (75) des langgestreckten Elements (42) in seinen/aus seinem Innenraum (73) umfasst, um einen Durchgang zu bilden, der wenigstens an einem Klemmbereich (76), welcher durch die Platte (84) und die untere Anlageseite (71) teilweise begrenzt ist, ausmündet.

11. Implantierbare Vorrichtung (40) nach einem der Ansprüche 1 bis 10, **dadurch gekennzeichnet, dass** die erste (51) und die zweite (52) Gabel entgegengesetzt sind.

12. Implantierbare Vorrichtung (2, 40) nach einem der Ansprüche 1 bis 11, **dadurch gekennzeichnet, dass** die erste (9, 51) und die zweite (10, 52) Gabel in parallelen Ebenen (p1, p2, p5, p6) liegen.

13. Implantierbare Vorrichtung (2, 40) nach einem der Ansprüche 1 bis 12, **dadurch gekennzeichnet, dass** das Mittel zum festen Verbinden (8, 13, 50) aus einem oder mehreren Materialien besteht, das/die aus der Liste, umfassend Titan, rostfreien Stahl, eine Titan-Nickel-Legierung, Polyetheretherketon (PEEK), PEK (Polyetherketon), Kobalt-Chrom, Polyamid 6,6, Polyamid 6, Polyethylenterephthalat, ausgewählt ist/sind.

14. Set (1) für eine implantierbare Vorrichtung, umfassend eine implantierbare Vorrichtung (2, 40) nach einem der Ansprüche 1 bis 13 und ein Werkzeug zum Greifen (3) des Mittels zum festen Verbinden (8, 13, 40), umfassend einen Stab (4), der an seinem Ende (3a) mit ersten Befestigungselementen (5) versehen ist, welche geeignet sind, mit zweiten Befestigungselementen (12, 23), die von dem Mittel zum festen Verbinden (8, 13, 40) getragen sind, zusammenzuwirken.

## Claims

1. An implantable device (2, 40), in particular to correct at least one vertebral level, optionally with fusion of said level, comprising:
a. a rod (6, 41) having an outer radius (Rt) and a circumference (Ct) determined at an area (z) along its length (Lₜ);
b. a flat elongate element (7, 42);
c. a device (8, 13, 50) to secure said elongate element (7, 42) in a position partly encasing said area (z);
**characterized in that** said securing device (8, 13, 50) comprises a first fork (9, 14, 51) and a second fork (10, 15, 52) having a first general partly annular shape of inner radius ri1 and ri2 respectively, **in that** said first (9, 14, 51) and second forks (10, 15, 52) comprise partly annular lower bearing surfaces (9a, 10a, 51a, 52a) respectively delimiting first (9b, 51b) and second (10b, 52b) portions of circumference greater than one half of the circumference (Ct), **in that** said securing device (8, 13, 50) comprises a shoulder part (11, 24, 60) arranged relative to said lower bearing surfaces (9a, 10a, 51a, 52a) of the first and second forks (9, 14, 51, 10, 15, 52) so as to form a passage for said elongate element in said encasing position between said forks and said shoulder part, and **in that** the first (9, 14, 51) and second (10, 15, 52) forks and their inner radii ri1 and ri2 are determined in relation to the outer radius Rt of the rod (6, 41) so that the press-fitting of the first (9, 14, 51) and second (10, 15, 52) forks either side of the elongate element (7, 42) partly encasing said rod (6, 7) causes deformation thereof and then their securing to said rod (6, 7).

2. The implantable device (2, 40) according to claim 1, **characterized in that** said shoulder part (11, 24, 60) is recessed relative to said lower bearing surfaces (9a, 10a, 51a, 52a), by a distance hi and h2 respectively so as to form a passage for said elongate element (7, 42).

3. The implantable device (40) according to either of claims 1 and 2, **characterized in that** the securing device (50) comprises a piece (70) having a lower bearing surface (71) capable of receiving at least one portion of said elongate element (42), and clamping device (80) which can be actuated in translation and arranged relative to said lower bearing surface (71) so that the movement in translation of said clamping device (80) allows the clamping of at least one portion of said elongate element (42) against said lower bearing surface (71).

4. The implantable device (2, 40) according to any one of claims 1 to 3, **characterized in that** the shoulder part (11, 24, 60) spaces apart the first fork (9, 14, 51) and the second fork (10, 15, 52) by a distance (d1, d2, d3) equal to or greater than the width (I, I1) of said flat elongate element (7, 42).

5. The implantable device (40) according to either of claims 3 and 4, **characterized in that** the piece (70) comprises a tapped hole (72), and the clamping device (80) comprise a screw (81) having a threaded portion (83) able to cooperate with said tapped hole (72), and **in that** said clamping device (80) further comprises a plate (84) and connecting device (85) ensuring a link between said screw (81) and the plate (84) and arranged so that the setting in rotation of said screw (81) causes the movement in translation of said plate (84) to clamp at least one portion of said elongate element (42) against the lower bearing surface (71).

6. The implantable device (40) according to claim 5, **characterized in that** the screw (81) at its proximal end (81a) comprises a housing (82) and **in that** the connecting device (85) comprises at least one deformable portion (86) able to be deformed so that it can be inserted in said housing (82) and to recover its non-deformed shape for securing to said housing (82).

7. The implantable device (40) according to either of claims 5 and 6, **characterized in that** the connecting device (85) is secured to said plate (84).

8. The implantable device (40) according to any one of claims 3 to 7, **characterized in that** the piece (70) is of parallelepiped shape, in particular rectangular parallelepiped shape comprising side surfaces (70c, 70d, 70e, 70f), and particularly opposite-lying upper (70a) and lower (70b) surfaces, said first (51) and second (52) forks projecting along a first side surface (70c) selected from among said side surfaces (70c, 70f) of the piece (70).

9. The implantable device (40) according to claim 8, **characterized in that** the shoulder part (60) is the upper surface (70a) of said piece (70).

10. The implantable device (40) according to any one of claims 3 to 9, **characterized in that** the piece (70) having an inner volume (73) comprises an inlet orifice (74) and an outlet orifice (75) in its inner volume (73) for said elongate element (42) so as to form a passage opening onto at least one clamping area (76) partly delimited by said plate (84) and the lower bearing surface (71).

11. The implantable device (40) according to any one of claims 1 to 10, **characterized in that** the first (51) and second (52) forks are in opposition.

12. The implantable device (2, 40) according to any one of claims 1 to 11, **characterized in that** the first (9, 51) and second (10, 52) forks lie in parallel planes (pi, p2, p5, p6).

13. The implantable device (2, 40) according to any one of claims 1 to 12, **characterized in that** said securing device (8, 13, 50) is in one or more materials selected from the list comprising: titanium, stainless steel, a titanium and nickel alloy, polyetheretherketone (PEEK), PEK (polyetherketone), chromium cobalt, polyamide 6-6, polyamide 6, ethylene polyterephthalate.

14. A kit (1) for implantable device comprising an implantable device (2, 40) according to any one of claims 1 to 13, and a grasping tool (3) for said securing device (8, 13, 40) comprising a bar (4) provided at its end (3a) with first attaching members (5) able to cooperate with second attaching members (12, 23) supported by said securing device (8, 13, 40).
